# DEMANDE DE BREVET EUROPEEN

(11) **EP 0 881 284 A1**
(43) Date de publication de la demande: **02.12.1998**
(21) Numéro de dépôt: 98401109.8
(22) Date de dépôt: 07.05.1998
(51) Int. Cl.: C12N 1/20, C12Q 1/10, C12Q 1/04

(54) **Milieu de culture pour la détection spécifique des entérobactéries, procédé pour son obtention et son utilisation**

(30) Priorité: 13.05.1997 FR 9705812
(71) Demandeur: Société La Biochimie Appliquée - SO.LA.BI.A., 93500 Pantin (FR)
(72) Inventeur: Perry, David, 60000 Beauvais (FR); Quiring, Christophe, 60000 Beauvais (FR)
(74) Mandataire: Nevant, Marc

(57) **Abrégé**

L'invention concerne un milieu de culture gelosé pour la détection spécifique de micro-organismes tel que les entérobactéries, qui comprend un substrat nutritif pour lesdits micro-organismes, au moins un composé chromogène ou fluorogène susceptible d'être hydrolysé par une enzyme de type estérase et/ou un composé chromogène ou fluorogène susceptible d'être hydrolysé par une enzyme de type glycosidase, et au moins un inhibiteur des micro-organismes non recherchés, le(s)dit(s) composé(s) chromogène(s) ou fluorogène(s) étant sous forme de complexe d'inclusion avec une cyclodextrine.

## Description

L'invention concerne le domaine du diagnostic. Plus précisément, l'invention concerne un milieu de culture pour la détection, le dénombrement et l'identification de micro-organismes tels que les entérobactéries. L'invention concerne aussi un procédé pour l'obtention de ce milieu et ses applications.

L'analyse microbiologique des aliments, des produits pharmaceutiques et cosmétiques, des échantillons cliniques et de tout prélèvement biologique en général, est intégrée dans chaque programme de surveillance en matière d'hygiène.

Les principales méthodes de détection et d'identification des micro-organismes tels que les entérobactéries sont basées sur la mise en évidence d'une ou plusieurs activités enzymatiques caractéristiques de ces bactéries. Ces méthodes sont souvent basées sur la fermentation de substrats et la production d'acide ou d'autres sous-produits. Les activités caractéristiques sont mises en évidence à l'aide d'indicateurs de pH ou par complexation avec des sels minéraux. Depuis quelques années ces méthodes mettent en oeuvre des substrats chromogènes et fluorogènes définis, qui permettent la visualisation de l'actvité enzymatique recherchée.

Ainsi, la demande de brevet GB-A-2 050 418 décrit par exemple un test pour détecter les bactéries du genre *Salmonella*, *Klebsiella, E. coli, Enterobacter* ou *Serratia* et les différencier de celles du genre *Proteus* ou *Providencia.* Dans un premier temps, on prépare un milieu de culture gélosé contenant tous les ingrédients nécessaires à la croissance de ces bactéries. Dans un second temps, on impreigne un disque en papier avec un substrat enzymatique synthétique sous la forme d'un ester ayant une chaîne aliphatique contenant de 7 à 10 atomes de carbone et éventuellement un substrat glucoside et/ou un substrat galactoside et on dépose à la surface du disque une colonie de bactéries isolées sur le milieu gélosé sus-mentionné. On procède enfin à la détection visuelle des bactéries en ajoutant un sel de diazonium à la surface du disque en papier.

Selon un autre procédé, décrit dans la demande de brevet FR-A-2 671 100, on effectue la détection des *Salmonella* en préparant tout d'abord un milieu gélosé comprenant un substrat nutritif métabolisé par lesdites bactéries, un composé chromogène ou fluorogène, susceptible d'être hydrolysé par la β-galactosidase, de l'acide glucuronique ou un sel de celui-ci et un indicateur de pH. On ensemence ensuite une boîte de Petri remplie de ce milieu avec un échantillon à analyser et on détermine la présence ou non des *Salmonella* en fonction de la coloration des colonies formées après 24 h d'incubation.

Un autre procédé pour la détection des *Salmonella*, décrit dans la demande de brevet FR-A-2 697 028, consiste à préparer un milieu de culture comprenant, outre un milieu de culture pour lesdites bactéries, une combinaison d'un composé chromogène lié à un acide gras en C₇-C₁₀ et d'un détergent favorisant l'incorporation du composé chromogène, à savoir un sel de dérivé acide carboxylique - polyols du cholane. Ce milieu est versé dans une boîte de Petri qui est alors ensemencée avec l'échantillon à analyser. La détection des *Salmonella* se fait après 24 h d'incubation en fonction de la coloration des colonies formées.

Ces différents documents ne présentent pas d'exemple de dénombrement d'entérobactéries. Les étapes de pré-incubation des méthodes qui y sont décrites augmentent le nombre de cellules d'entérobactéries jusqu'à un seuil détectable.

La formulation de milieux de culture gélosés à base d'acide gras se heurte en outre à plusieurs difficultés techniques:
- les dérivés d'acide gras sont souvent peu ou pas hydrosolubles;
- les détergents et solvants utilisés ainsi que les dérivés d'acide gras sont souvent toxiques pour les micro-organismes;
- les dérivés d'acide gras sont souvent thermolabiles et par conséquent ils ne peuvent pas être incorporés dans un milieu de culture destiné à la stérilisation à l'autoclave;
- les acides gras peuvent être incompatibles avec les autres ingrédients du milieu de culture.

La formulation de milieux de culture gélosés à base de dérivé chromogène d'acide gras se heurte également au problème de la spécificité de la réaction recherchée.

Les micro-organismes tels que les bactéries possèdent en effet des activités enzymatiques caractéristiques. Par exemple, les bactéries du genre *Pseudomonas, Salmonella, Enterobacter* et *Klebsiella* possèdent une activité estérase caractéristique. Ces deux dernières bactéries possèdent également une activité β-glucosidase caractéristique. On comprend donc qu'il est difficile de détecter de manière spécifique une bactérie donnée lorsque celle-ci possède une ou plusieurs activités en commun avec une ou plusieurs autres bactéries.

Les méthodes de détection de l'art antérieur ne permettent pas de détecter de manière spécifique l'activité caractéristique des bactéries recherchées
- soit parce que, dans le cas du document FR-A- 2 671 100, la fermentation d'acide glucuronique est aussi caractéristique des genres *Escherichia* et *Enterobacter* et certaines souches de ces genres ne produisent pas de galactosidase ; que les genres *Morganella* et *Shigella* fermentent l'acide glucuronique et que la plupart des souches de ces genres ne produisent pas de galactosidase ; ou que la galactosidase est aussi caractéristique de *Salmonella diarizonae* ; ce qui conduit dans les deux premiers cas à des résultats faux positifs et dans le dernier cas à des résultats faux négatifs;
- soit parce que, dans le cas du document FR-A- 2 697 028, le milieu de détection utilisé est basé sur le principe de détection de l'estérase des *Salmonella* ; que les micro-organismes non-*Salmonella* qui possèdent l'activité estérase et qui utilisent le cellobiose comme substrat auront une couleur contrastée; et que les micro-organismes non-*Salmonella* possédant l'activité estérase (tel que *Pseudomonas)* qui n'utilisent pas le cellobiose comme substrat, seront confondus avec *Salmonella.*

Il a maintenant été trouvé d'une manière inattendue que l'incorporation de substrats chromogènes ou fluorogènes dans un milieu de culture gélosé au moyen de cyclodextrines permet la détection spécifique de micro-organismes tels que les entérobactéries à l'aide dudit milieu de culture.

Ainsi, un premier objet de l'invention concerne un milieu de culture gélosé qui permet la détection spécifique de micro-organismes tels que les entérobactéries.

Un autre objet de l'invention concerne un milieu de culture gélosé qui permet le dénombrement d'un genre de micro-organismes tels que les entérobactéries dans un échantillon donné.

Ces objets sont atteints par le milieu de culture gélosé pour la détection spécifique de micro-organismes tels que les entérobactéries conforme à l'invention, qui comprend un substrat nutritif pour lesdits micro-organismes, au moins un composé chromogène ou fluorogène susceptible d'être hydrolysé par une enzyme de type estérase et/ou un composé chromogène ou fluorogène susceptible d'être hydrolysé par une enzyme de type glycosidase, et au moins un inhibiteur des micro-organismes non recherchés, notamment les bactéries Gram⁻ et/ou Gram⁺, le(s)dit(s) composé(s) chromogène(s) ou fluorogène(s) étant sous forme de complexe d'inclusion avec des cyclodextrines.

Le milieu de culture gélosé selon l'invention permet la détection spécifique de différents types de micro-organismes, notamment des entérobactéries du genre *Enterobacter, Escherichia, Klebsiella, Salmonella, Serratia* et *Yersinia.*

Ledit milieu de culture gélosé comprend donc, outre un substrat nutritif pour au moins un micro-organisme donné, notamment une entérobactérie donnée, au moins un inhibiteur des micro-organismes non recherchés, et un ou plusieurs composés chromogènes ou fluorogènes susceptibles d'être hydrolysés par une enzyme de type estérase ou glycosidase. Par enzyme de type glycosydase, on entend au sens de la présente invention une enzyme capable de rompre des liaisons entre un substrat chromogène ou fluorogène et des saccharides ou oligosaccharides tels que les saccharides ou oligosaccharides de glucose, galactose, rhamnose, fucose, xylose, mannose, arabinose, d'acide glucuronique, de galactosamine ou de glucosamine.

De préférence, ledit milieu comprend au moins un composé chromogène ou fluorogène susceptible d'être hydrolysé par une enzyme de type estérase et au moins un composé chromogène ou fluorogène susceptible d'être hydrolysé par une enzyme de type glycosidase.

Les composés chromogènes ou fluorogènes sont présents dans le milieu de culture gélosé sous forme de complexe d'inclusion avec des cyclodextrines.

A titre d'exemple de cyclodextrines qu'il est possible d'utiliser avantageusement dans le cadre de l'invention, on peut citer l'α-cyclodextrine, la β-cyclodextrine, la γ-cyclodextrine, la méthylcyclodextrine, la diméthycyclodextrine, la triméthylcyclodextrine, I'hydroxypropylcyclodextrine, les β-cyclodextrines méthylées de façon aléatoire ou un mélange de ces composés.

Avantageusement, on utilise les cyclodextrines à une concentration comprise entre environ 0,5 mg/l et environ 30 g/l, de préférence entre environ 20 mg/l et environ 10 g/l.

Les composés chromogènes ou fluorogènes conformes à l'invention sont de deux types : ceux qui sont susceptibles d'être hydrolysés par une enzyme de type estérase et ceux qui sont susceptibles d'être hydrolysés par une enzyme de type glycosidase.

Les premiers sont généralement constitués par un substrat chromogène ou fluorogène lié à un acide gras à chaîne courte ou à chaîne longue, notamment un acide gras en C₇-C₁₀, de préférence l'acide caprylique (C₈).

Les seconds sont généralement constitués par un substrat chromogène ou fluorogène lié à un saccharide ou un oligosaccharide de glucose, galactose, rhamnose, fucose, xylose, mannose, arabinose, d'acide glucuronique, de galactosamine ou glucosamine, de preférence un glucoside ou un galactoside.

On utilise de préférence chaque composé chromogène ou fluorogène à une concentration comprise entre environ 0,5 mg/l et environ 1 g/l, avantageusement entre environ 10 mg/l et 500 mg/l.

Les substrats chromogènes ou fluorogènes du premier et du second types sont ceux utilisés de manière conventionnelle pour la détection des micro-organismes tels que les entérobactéries. A titre d'exemple, on peut citer le 5-bromo-4-chloro-3-indole, le 5-bromo-6-chloro-3-indole, le 6-chloro-3-indole, le méthyl-3-indole, le 5-bromo-3-indole, le 6-fluoro-3-indole, le 5-iodo-3-indole, la 4-méthylumbelliférone, la 8-hydroxyquinoléine, l'amidométhylcoumarine, la fluorescéine ou leurs dérivés, le 5-bromo-4-chloro-3-indole et le 5-bromo-6-chloro-3-indole étant préférés.

Selon une autre caractéristique, le milieu de culture gélosé conforme à l'invention contient au moins un inhibiteur des micro-organismes non recherchés, notamment des bactéries Gram⁻ et/ou Gram⁺. Cet inhibiteur, en combinaison avec le complexe d'inclusion entre d'une part la cyclodextrine et d'autre part le(s) composé(s) chromogène(s) ou fluorogène(s) permet d'obtenir un milieu de culture gélosé tout à fait spécifique d'un genre de micro-organismes, notamment d'un genre d'entérobactéries. On peut notamment utiliser à cet effet un ou plusieurs composés choisis parmi la bile et les sels biliaires ; un ester d'acide gras tel qu'un alkylsulfate de sodium, notamment le laurylsulfate de sodium ; l'acide cholique ou un de ses dérivés comme par exemple le chénodésoxycholate de sodium ou le désoxycholate de sodium ; un antibiotique comme par exemple un antibiotique de la famille des céphalosporines, la kanamycine, la novobiocine, la vancomycine, le céfamandole, la néomycine ou la tobramycine; un colorant tel que le vert malachite, le vert brillant ou le cristal violet.

Avantageusement, on utilise la bile ou les sels biliaires à une concentration comprise entre environ 100 mg/l et environ 15 g/l ; l'ester d'acide gras à une concentration comprise entre environ 100 mg/l et environ 2 g/l ; l'acide cholique et ses dérivés à une concentration comprise entre environ 100 mg/l et environ 3 g/l ; l'antibiotique à une concentration comprise entre environ 10 mg/l et environ 200 mg/l ; et le colorant à une concentration comprise entre environ 1 mg/l et environ 100 mg/l.

Le milieu de culture gélosé conforme à l'invention est particulièrement adapté à la détection des *Salmonella.* Dans ce cas, ledit milieu comprend de préférence, outre un milieu nutritif pour les *Salmonella,* un substrat chromogène ou fluorogène lié à un acide gras en C₇-C₁₀, un substrat chromogène ou fluorogène lié à un saccharide, et au moins un inhibiteur des bactéries Gram⁻ et/ou Gram⁺, lesdits composants dudit milieu étant tels que définis précédemment. Avantageusement, on utilise le 5-bromo-6-chloro-3-indolyl-caprylate comme substrat chromogène ou fluorogène lié à l'acide gras en C₇-C₁₀, et le 5-bromo-4-chloro-3-indolyl-glucoside comme substrat chromogène ou fluorogène lié au saccharide.

Selon un mode de réalisation particulier de l'invention, le milieu de culture gélosé pour la détection spécifique des *Salmonella* peut également comprendre un inducteur de la synthèse de l'estérase comme par exemple le citrate de fer et/ou un inducteur de la synthèse de la β-glucosidase comme par exemple un alkylglucoside.

Le milieu de culture gélosé conforme à l'invention est préparé en incorporant les composés chromogènes ou fluorogènes dans ledit milieu à l'aide de cyclodextrines.

Ainsi, selon un autre aspect, l'invention concerne un procédé d'obtention d'un milieu de culture gélosé pour la détection spécifique de micro-organismes tels que les entérobactéries, contenant au moins un composé chromogène ou fluorogène susceptible d'être hydrolysé par une enzyme de type estérase et/ou un composé chromogène ou fluorogène susceptible d'être hydrolysé par une enzyme de type glycosidase, qui consiste à incorporer les composés chromogènes ou fluorogènes dans le milieu au moyen de cyclodextrines. Avantageusement, le procédé conforme à l'invention consiste à
(i) mélanger en milieu sec ou en milieu aqueux les composés chromogènes ou fluorogènes avec des cyclodextrines ; et
(ii) incorporer ledit mélange dans un milieu de culture gélosé donné.

Selon une variante, le mélange de l'étape (i) s'effectue en milieu solvant organique. A titre de solvant adéquat, on peut citer les cétones telles qu'acétone ou butanone ; les alcools tels que méthanol, éthanol ou propanol ; le diméthylformamide ; le diméthylsulfoxyde ; ou un mélange de ces composés. On peut également utiliser avantageusement un mélange d'eau et d'un ou plusieurs solvants organiques tels que définis ci-dessus. Lorsqu'on utilise un solvant, les composés chromogènes ou fluorogènes sont généralement dissous dans ledit solvant afin d'obtenir une solution claire et limpide. Cette solution est ensuite ajoutée à une solution aqueuse de cyclodextrines et l'ensemble est agité mécaniquement. On obtient ainsi une solution de composés chromogènes ou fluorogènes sous forme de complexe d'inclusion avec des cyclodextrines. La solution de cyclodextrines incluant les composés chromogènes ou fluorogènes est alors ajoutée au milieu de culture gélosé de manière à obtenir une concentration finale pour chaque composé chromogène ou fluorogène d'environ 0,5 mg/l à environ 1 g/l dans le milieu de culture gélosé. L'ensemble est alors agité de manière à bien répartir la solution de cyclodextrines et obtenir un milieu de culture gélosé homogène.

Le milieu de culture gélosé conforme à l'invention permet la détection spécifique ainsi que le dénombrement des micro-organismes tels que les entérobactéries, notamment les *Salmonella,* dans un échantillon à analyser.

En effet, les colonies de tous les genres d'entérobactéries cultivées sur un milieu contenant des composés chromogènes développent des colorations ou des fluorescences caractéristiques en fonction de leur(s) activité(s) enzymatique(s). Les colorations sont visibles à l'oeil nu et les fluorescences sont visibles sous un éclairage ultraviolet.

Par exemple, cultivées sur un milieu contenant des composés chromogènes, les colonies de *Salmonella,* possédant une activité estérase, produisent une coloration caractéristique. La plupart des bactéries non *Salmonella* sont inhibées. Les autres bactéries cultivant sur le milieu possèdent une autre activité caractéristique et leurs colonies développent une coloration contrastée à celle de *Salmonella,* cette coloration étant caractéristique de ces bactéries.

Ainsi, selon un autre aspect, l'invention concerne une méthode de détection spécifique des micro-organismes tels que les entérobactéries, notamment les *Salmonella,* dans un échantillon à analyser, qui consiste à ensemencer le milieu de culture gélosé défini ci-dessus avec ledit échantillon puis à repérer les colonies présentant la coloration caractéristique des bactéries recherchées.

L'invention concerne également une méthode pour le dénombrement des micro-organismes tels que les entérobactéries, notamment les *Salmonella,* dans un échantillon à analyser. Le milieu de culture défini ci-dessus permet en effet de déterminer directement le nombre de bactéries telles que les *Salmonella* présentes dans un échantillon donné : sa spécificité permet le dénombrement des bactéries par comptage des colonies présentant la coloration caractéristique recherchée, rendant les étapes de confirmations inutiles.

Le milieu de culture gélosé incorporant le(s) composé(s) chromogène(s) ou fluorogène(s) peut être distribué dans des boîtes de Petri stériles et ensemencé suivant l'une ou l'autre des techniques couramment utilisées en bactériologie : soit en surface soit dans la masse.

Un exemple de milieu de culture gélosé conforme à l'invention consiste à réaliser le mélange 5-bromo-6-chloro-3-indolyl-caprylate / 5-bromo-4-chloro-3-indolyl-glucoside / β-cyclodextrine méthylée de façon aléatoire et à l'incorporer dans un milieu de culture comme décrit ci-dessus. En présence de ces composés chromogènes, on observe les colorations suivantes :
- les bactéries possédant une activité estérase hydrolysent le 5-bromo-6-chloro-3-indolyl-caprylate et dévelopent des colonies rouges;
- les bactéries possédant une activité β-glucosidase hydrolysent le 5-bromo-4-chloro-3-indolyl-glucoside et développent des colonies bleues;
- les bactéries possédant les deux activités enzymatiques produisent des colonies bleu-violet;
- les bactéries dépourvues de ces deux activités sont blanchâtres.

Le milieu de culture gélosé conforme à l'invention peut être utilisé avantageusement dans le domaine du diagnostic alimentaire, pharmaceutique, médical, cosmétique et pour les analyses de l'environnement. Il est tout particulièrement adapté à la détection des entérobactéries dans les aliments, notamment pour les contrôles nécéssitant la détéction des *Salmonella.*

L'invention sera mieux comprise à l'aide des exemples ci-après, donnés à titre illustratif.

### EXEMPLE 1 : Milieu pour la détection des Salmonella

On prépare suivant les techniques bien connues de l'homme du métier le milieu de culture gélosé de composition suivante:

| **Apport** | **Composition** | **Quantité** |
|---|---|---|
| Base nutritif | Hydrolysat de protéine (peptone) | 1 à 20 g |
| | Pyruvate | 500 mg à 3 g |
| | Chlorure de Sodium | 1 à 10 g |
| | Dihydrogénophosphate de potassium | 2 à 15 g |
| | Hydrogénophosphate de sodium | 1 à 5 g |
| | Agar | 13 à 22 g |
| Base chromogène | 5-bromo-6-chloro-3-indolyl-caprylate | 0,5 mg à 1 g |
| | 5-bromo-4-chloro-3-indolyl-glucoside | 0,5 mg à 1 g |
| | Cyclodextrines | 0,5 à 30 g |
| Inducteurs | Méthyl-glucoside | 0,25 à 4 g |
| | Citrate de fer | 0,5 à 5 g |
| Agents inhibiteurs | Sels biliares | 1 à 13 g |
| | Céphalosporine | 5 à 25 mg |
| | Eau qsp | 1 l |

Ce milieu, appelé milieu BKD1 dans ce qui suit, se présente avantageusement sous forme déshydratée, auquel cas il doit être réhydraté de manière conventionnelle avant utilisation.

### EXEMPLE 2 : Spécificité du milieu BKD1

On a ensemencé en stries selon la méthode des quadrants, dans des boîtes de Petri, le milieu BKD1 avec diverses souches de bactéries (cf Tableau 1).

Après incubation à 37° C pendant 16 à 24 heures, on observe la coloration des colonies. La coloration des colonies permet de distinguer les *Salmonella* des autres bactéries.

**Tableau 1**

| Micro-organismes | Croissance | Aspect des colonies |
|---|---|---|
| *Salmonella typhimurium* ATCC 14028 | bonne à excellente | rouge |
| *Salmonella enteritidis* ATCC 13076 | bonne à excellente | rouge |
| *Salmonella virchow* BDCC* 28 | bonne à excellente | rouge |
| *Salmonella typhi* BDCC* 358 | bonne | rouge |
| *Escherichia coli* ATCC 25922 | bonne | blanchâtre |
| *Enterobacter cloacae* ATCC 13047 | bonne à excellente | bleu à violet |
| *Enterobacter aerogenes* ATCC 13048 | bonne à excellente | bleu à violet |
| *Klebsiella pneumoniae* ATCC 13883 | bonne | bleu à violet |
| *Shigella sonnei* ATCC 29930 | bonne | blanchâtre |
| *Pseudomonas aeruginosa* ATCC 9027 | inhibée | - |
| *Staphylococcus aureus* ATCC 25923 | inhibée | - |
| *Enterococcus faecalis* ATCC 29912 | inhibée | - |

| | | |
|---|---|---|
| * la collection BDCC est une collection interne de la Demanderesse | | |

Par ailleurs, l'examen des résultats présentés aux Tableaux 2 et 3 ci-après démontre la grande polyvalence du milieu BKD1 pour l'analyse de souches variées de *Salmonella* et d'autres entérobactéries et aussi l'extrême spécificité de ce milieu.

**Tableau 2 :**

| Coloration des souches de *Salmonella*. | | |
|---|---|---|
| SOUCHES | n | n rouge |
| *Salmonella enterica* subsp. *enterica* sérotype Enteritidis | 19 | 19 |
| *Salmonella enterica* subsp. *enterica* sérotype Typhimurium | 20 | 20 |
| *Salmonella enterica* subsp. *enterica* sérotype Virchow | 16 | 16 |
| *Salmonella enterica* subsp. *enterica* sérotypes divers | 121 | 121 |
| *Salmonella enterica* subsp. *arizonae* | 2 | 2 |
| *Salmonella enterica* subsp. *diarizonae* | 19 | 19 |
| *Salmonella enterica* subsp. *houtenae* | 15 | 15 |
| *Salmonella enterica* subsp. *salamae* | 11 | 11 |
| **TOTAL** | **192** | **192** |
| (n = nombre) | | |

**Tableau 3 :**

| Croissance et coloration des souches de bactéries non-*Salmonella* | | | | | |
|---|---|---|---|---|---|
| | n | n rouge | n bleu-violet | n blanchâtre | n inhibée |
| *Citrobacter spp.* | 1 7 | 0 | 0 | 1 5 | 2 |
| Diverses Gram - | 13 | 0 | 0 | 10 | 3 |
| *E. coli* | 112 | 0 | 0 | 101 | 11 |
| *Enterobacter spp.* | 29 | 0 | 29 | 0 | 0 |
| *Enterococcus* spp. | 20 | 0 | 0 | 0 | 20 |
| *Hafnia* spp. | 8 | 0 | 0 | 2 | 6 |
| *Klebsiella* spp. | 12 | 0 | 10 | 0 | 2 |
| *Proteus* spp. | 11 | 0 | 0 | 9 | 2 |
| *Providencia* spp. | 2 | 0 | 0 | 2 | 0 |
| *Pseudomonas* spp. | 26 | 0 | 0 | 0 | 26 |
| *Serratia* spp. | 4 | 0 | 0 | 2 | 2 |
| *Shigella* spp. | 9 | 0 | 0 | 3 | 6 |
| *Staphylococcus* spp. | 31 | 0 | 0 | 0 | 31 |
| *Streptococcus* spp. | 21 | 0 | 0 | 0 | 21 |
| *Yersinia* spp. | 5 | 0 | 0 | 1 | 4 |
| **TOTAL** | **320** | **0** | **39** | **145** | **136** |

### EXEMPLE 3 : Dénombrement des Salmonella

Divers échantillons alimentaires stérilisés sont artificiellement contaminés puis homogénéisés dans de l'eau peptonée tamponnée et le milieu BKD1 est ensemencé directement avec ces échantillons, sans incubation prolongée préalable, par les méthodes bien connues de l'homme du métier. Après 16 à 24 h d'incubation à 37°C, le nombre et la coloration des colonies sont relevés et les colonies sont identifiées. Parallèlement, le nombre d'unités formant colonie (UFC) de *Salmonella* est déterminé par cytométrie de flux. Les valeurs obtenues sont également comparées avec celles établies à l'aide d'un milieu de culture gélosé non séléctif du type caséine-soja. Les résultats obtenus sont présentés dans le Tableau 4.

**Tableau 4**

| Echantillon alimentaire | n *Salmonella* présentes ¹ (UFC/g) | n *Salmonella* présentes ² (UFC/g) | n colonies rouges *(Salmonella* confirmées) ³ |
|---|---|---|---|
| 1 | 25 | 15 | 21 |
| 2 | 160 | 124 | 148 |
| 3 | 5800 | 3826 | 5950 |
| 4 | 12340 | 8763 | 12620 |

| | | | |
|---|---|---|---|
| ¹ : détermination par cytométrie de flux | | | |
| ² : détermination sur gélose non sélective caséine-soja | | | |
| ³ : détermination sur le milieu BKD1 | | | |

### EXEMPLE 4 : Détection et dénombrement des Escherichia coli

On a procédé comme aux exemples 2 et 3, excepté que le milieu de culture gélosé contenait en outre 0,5 à 500 mg de 4-méthylumbelliféryl-glucuronate. Ce milieu, ci-après appelé milieu BKD2, a été ensemencé dans des boîtes de Petri, en stries selon la méthode des quadrants, avec diverses souches de bactéries (Tableau 5). Après incubation à 37° C pendant 16 à 24 heures, on observe la coloration et la fluorescence des colonies. La coloration et la fluorescence des colonies permet de distinguer les *Escherichia coli* des autres bactéries.

**Tableau 5**

| **Micro-organismes** | **Croissance** | **Aspect des colonies** |
|---|---|---|
| *Salmonella typhimurium* ATCC 14028 | bonne à excellente | rouge |
| *Salmonella enteritidis* ATCC 13076 | bonne à excellente | rouge |
| Salmonella *virchow* BDCC* 28 | bonne à excellente | rouge |
| *Salmonella typhi* BDCC* 358 | bonne | rouge |
| *Escherichia coli* ATCC 25922 | bonne à excellente | fluorescente |
| *Escherichia coli* ATCC 8739 | bonne à excellente | fluorescente |
| *Enterobacter cloacae* ATCC 13047 | bonne à excellente | bleu à violet |
| *Enterobacter aerogenes* ATCC 13048 | bonne à excellente | bleu à violet |
| *Klebsiella pneumoniae* ATCC 13883 | bonne | bleu à violet |
| *Shigella* flexneri ATCC 12022 | bonne | blanchâtre |
| *Pseudomonas aeruginosa* ATCC 9027 | inhibée | - |
| *Staphylococcus aureus* ATCC 25923 | inhibée | - |
| *Enterococcus faecalis* ATCC 29212 | inhibée | - |

| | | |
|---|---|---|
| * : la collection BDCC est une collection interne à la Demanderesse | | |

Divers échantillons alimentaires stérilisés sont artificiellement contaminés, puis homogénéisés dans de l'eau peptonée tamponnée. Le milieu est ensemencé directement avec ces échantillons, sans incubation préalable, par les méthodes bien connues de l'homme du métier. Après 16 à 24 heures d'incubation à 37° C, le nombre, la coloration et la fluorescence des colonies sont relevées et les colonies identifiées. Parallèlement, le nombre d'UFC d'*Escherichia coli* est déterminé par cytométrie de flux. Les valeurs obtenues sont également comparées avec celles établies à l'aide d'un milieu gélosé non sélectif de type gélose caséine-soja. Les résultats obtenus sont présentés dans le tableau 6.

**Tableau 6**

| Echantillon alimentaire | n *E. coli* présentes ¹ (UFC/g) | n *E. coli* présentes ² (UFC/g) | n colonies fluorescentes ³ *(E. coli* confirmées) |
|---|---|---|---|
| 1 | 18 | 14 | 16 |
| 2 | 215 | 194 | 202 |
| 3 | 2586 | 2105 | 2343 |
| 4 | 33855 | 31067 | 31504 |

| | | | |
|---|---|---|---|
| ¹ : détermination par cytométrie de flux | | | |
| ² : détermination sur gélose non sélective caséine-soja | | | |
| ³ : détermination sur le milieu BKD2 | | | |

Comme on peut le constater, le milieu de culture gélosé conforme à l'invention permet de déterminer de manière spécifique et quantitative le nombre de bactéries telles que *Salmonella* ou *Escherichia coli* présentes dans un aliment, avec une précision égale ou supérieure à celle (10 %) couramment acceptée pour ce type de méthode.

## Revendications

1. Milieu de culture gelosé pour la détection spécifique de micro-organismes tel que les entérobactéries, qui comprend un substrat nutritif pour lesdits micro-organismes, au moins un composé chromogène ou fluorogène susceptible d'être hydrolysé par une enzyme de type estérase et/ou un composé chromogène ou fluorogène susceptible d'être hydrolysé par une enzyme de type glycosidase, et au moins un inhibiteur des micro-organismes non recherchés, le(s)dit(s) composé(s) chromogène(s) ou fluorogène(s) étant sous forme de complexe d'inclusion avec une cyclodextrine.

2. Milieu de culture selon la revendication 1, dans lequel ledit composé chromogène ou fluorogène susceptible d'être hydrolysé par l'enzyme estérase est un substrat chromogène ou fluorogène lié à un acide gras en C₇-C₁₀.

3. Milieu de culture selon la revendication 1 ou 2, dans lequel ledit composé chromogène ou fluorogène susceptible d'être hydrolysé par l'enzyme glycosidase est un substrat chromogène ou fluorogène lié à un saccharide ou oligosaccharide.

4. Milieu de culture selon l'une des revendications 1 à 3, dans lequel ledit saccharide ou oligosaccharide est un saccharide ou oligosaccharide de glucose, galactose, rhamnose, fucose, xylose, mannose, arabinose, d'acide glucuronique, de galactosamine ou glucosamine.

5. Milieu de culture selon l'une des revendications 1 à 4, dans lequel ledit substrat chromogène ou fluorogène est choisi parmi le 5-bromo-4-chloro-3-indole, le 5-bromo-6-chloro-3-indole, le 6-chloro-3-indole, le méthyl-3-indole, le 5-bromo-3-indole, le 6-fluoro-3-indole, le 5-iodo-3-indole, la 4-méthyl-umbelliférone, la 8-hydroxyquinoléine, l'amidométhylcoumarine et la fluorescéine.

6. Milieu de culture selon l'une des revendications 1 à 5, dans lequel ledit inhibiteur des micro-organismes non recherchés est un inhibiteur des bactéries Gram⁻ et/ou Gram⁺.

7. Milieu de culture selon la revendication 6, dans lequel ledit inhibiteur des bactéries Gram⁻ et/ou Gram⁺ est choisi parmi la bile et les sels biliaires ; un ester d'acide gras ; l'acide cholique et ses dérivés ; un antibiotique ; et un colorant.

8. Milieu de culture selon l'une des revendications 1 à 7, dans lequel ladite cyclodextrine est choisie parmi l'a-cyclodextrine, la β-cyclodextrine, la γ-cyclodextrine, la méthylcyclodextrine, la diméthycyclodextrine, la triméthylcyclodextrine, l'hydroxypropyl-cyclodextrine, les β-cyclodextrines méthylées de façon aléatoire et un mélange de ces composés.

9. Milieu de culture selon l'une des revendications 1 à 8, qui comprend au moins un composé chromogène ou fluorogène susceptible d'être hydrolysé par une enzyme de type estérase et au moins un composé chromogène ou fluorogène susceptible d'être hydrolysé par une enzyme de type glycosidase

10. Milieu de culture selon la revendication 9, pour la détection spécifique des *Salmonella,* qui comprend un substrat nutritif pour lesdites bactéries, un substrat chromogène ou fluorogène lié à un acide gras en C₇-C₁₀, un substrat chromogène ou fluorogène lié à un glucoside et au moins un inhibiteur des bactéries Gram- et/ou Gram⁺, lesdits composés chromogènes ou fluorogènes étant sous forme de complexe d'inclusion avec une cyclodextrine.

11. Milieu de culture selon la revendication 10, dans lequel ledit substrat chromogène ou fluorogène est tel que défini à la revendication 5.

12. Milieu de culture selon la revendication 10 ou 11, dans lequel ledit inhibiteur des bactéries Gram⁻ et/ou Gram⁺ est tel que défini à la revendication 7.

13. Milieu de culture selon l'une des revendications 10 à 12, dans lequel ladite cyclodextrine est telle que définie à la revendication 8.

14. Procédé d'obtention d'un milieu de culture gélosé pour la détection spécifique de micro-organismes tels que les entérobactéries, contenant au moins un composé chromogène ou fluorogène susceptible d'être hydrolysé par une enzyme de type estérase et/ou un composé chromogène ou fluorogène susceptible d'être hydrolysé par une enzyme de type glycosidase, qui consiste à incorporer lesdits composés chromogènes ou fluorogènes dans ledit milieu au moyen de cyclodextrines.

15. Procédé selon la revendication 14, qui consiste à
(i) mélanger en milieu sec ou en milieu aqueux les composés chromogènes ou fluorogènes avec une cyclodextrine ; et
(ii) incorporer ledit mélange dans un milieu de culture gélosé donné.

16. Procédé selon la revendication 15, dans lequel l'étape (i) est effectuée en milieu eau/solvant organique.

17. Utilisation des cyclodextrines pour la préparation d'un milieu de culture gélosé pour la détection spécifique de micro-organismes tels que les entérobactéries.

18. Méthode de détection spécifique des micro-organismes tels que les entérobactéries, notamment des *Salmonella,* dans un échantillon à analyser, qui consiste à ensemencer le milieu de culture gélosé défini dans l'une des revendications 1 à 13 avec ledit échantillon puis à repérer les colonies présentant la coloration caractéristique des bactéries recherchées.

19. Application du milieu de culture gélosé selon l'une des revendications 1 à 13 au dénombrement des micro-organismes tels que les entérobactéries dans un échantillon à analyser.
